(19) Europäisches Patentamt
European Patent Office
Office européen des brevets

(11) Veröffentlichungsnummer: **0 414 171 B1**

(12) **EUROPÄISCHE PATENTSCHRIFT**

(45) Veröffentlichungstag der Patentschrift: **23.11.94**

(51) Int. Cl.5: **C12P 19/26**, C07H 5/06

(21) Anmeldenummer: **90115862.6**

(22) Anmeldetag: **18.08.90**

(54) **Verfahren zur enzymatischen Synthese galactosylierter Glycoprotein-Bausteine.**

(30) Priorität: **23.08.89 DE 3927801**

(43) Veröffentlichungstag der Anmeldung:
**27.02.91 Patentblatt 91/09**

(45) Bekanntmachung des Hinweises auf die Patenterteilung:
**23.11.94 Patentblatt 94/47**

(84) Benannte Vertragsstaaten:
**BE CH DE DK ES FR GB GR IT LI LU NL**

(56) Entgegenhaltungen:
**EP-A- 0 220 750**
**WO-A-87/05330**

**PATENT ABSTRACTS OF JAPAN, Band 11, Nr. 365 (C-460)[2812], 27. November 1987&NUM;**

**ARCHIVES OF BIOCHEMISTRY AND BIOPHYSICS, Band 153, Nr. 2, Dezember 1972; A.M. LESENEY et al., Seiten 831-836&NUM;**

**BIOCHEMICAL JOURNAL, Band 141, Nr. 1, Juli 1974; R.J. POLLITT et al., Seiten 141-146&NUM;**

(73) Patentinhaber: **HOECHST AKTIENGESELLSCHAFT**

**D-65926 Frankfurt (DE)**

(72) Erfinder: **Thiem, Joachim, Prof. Dr.**
**Leopardenstrasse 9**
**D-2000 Hamburg (DE)**
Erfinder: **Wiemann, Torsten**
**Englische Planke 6**
**D-2000 Hamburg (DE)**

**EP 0 414 171 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Glycoproteine spielen eine zentrale Rolle bei biologischen Erkennungsprozessen, wie der Tumorgenese, der bakteriellen und viralen Infektion, der Zell-Zell-Erkennung, dem Zellwachstum und der Zelldifferenzierung. Sie bilden die Grundlage der Blutgruppenklassifizierung und sind verantwortlich für die Internalisierung verschiedener makromolekularer Stoffe und Medikamente. Ein wichtiges Einsatzgebiet von Glycoproteinen ist daher z.B. die selektive Adressierung von Pharmaka an das Zielorgan sowie der Schutz von Medikamenten vor proteolytischem Abbau [N. Sharon, H. Lis, Chem. Eng. News 59 (13) (1981) 21; H. Schachter. Clinical Biochemistry 17, (1984) 3].

Glycoproteine werden je nach Art der Bindung zwischen Peptid- und Kohlenhydratteil in O-glycosidische und N-glycosidische Glycoproteine unterteilt. Letztere weisen als zentrale Verknüpfungseinheit nahezu ausschließlich eine Bindung von N-Acetylglucosamin mit Asparagin auf. Vor dem Hintergrund der genannten Funktionen von Glycoproteinen beansprucht die Synthese glycosylierter Aspartyl-N-acetylglucosamine besonderes Interesse.

Die Synthese solcher Glycoprotein-Bausteine mit Enzymen, die dem Biosyntheseweg dieser Substanzen in vitro folgen, hat den Vorteil der hohen Stereo- und Regioselektivität sowie der Vermeidung aufwendiger Schutzgruppenchemie, wodurch eine im Vergleich zur vielstufigen chemischen Synthese, meist gute Ausbeute gesichert wird.

Die Galactosyltransferase (EC 2.4.1.22) überträgt Galactose regio- und stereospezifisch auf N-Acetylglucosamin, wobei eine $\beta(1\rightarrow4)$-Bildung gebildet wird. Natürliche Substrate der Galactosyltransferase, sind Glucose, die jedoch nur in Gegenwart von $\alpha$-Lactalbumin von dem Enzym akzeptiert wird, sowie N-Glycoproteine mit N-Acetylglucosamin am terminalen Ende (WO-A-8 705 330).
Reaktionen des Enzyms mit nicht-natürlichen Substraten werden in den folgenden Veröffentlichungen beschrieben:

1. H.A. Nunez, R. Barker; Biochemistry 19, 489 (1980)
2. J. Thiem, W. Treder, T. Wiemann; Dechema Biotechnology Conferences, Vol. 2, 189 (1988), Ed.: D. Behrens, VCH-Verlagsgesellschaft, Weinheim
3. C. Augé, S. David, C. Mathieu, C. Gautheron; Tetrahedron Lett. 25, 1467 (1984)
4. U. Zehavi, M. Herchman; Carbohydr. Res. 128, 160 (1984)
5. U. Zehavi, S. Sadeh, M. Herchman; Carbohydr. Res. 124, 23 (1983)
6. U. Zehavi, M. Herchman; Carbohydr. Res. 133, 339 (1984)
7. C. Augé. C. Mathieu, C. Merienne; Carbohydr. Res. 151, 147 (1986)
8. M.M. Palcic, O.P. Srivastava, O. Hindsgaul; Carbohydr. Res. 159, 315 (1987)

Hierbei werden Saccharide oder niedere Oligosaccharide mit Glucose oder aber N-Acetylglucosamin am terminalen Ende mit Hilfe des Enzyms umgesetzt. Glykokonjugate aus Aminosäuren und Kohlenhydraten, insbesondere entsprechende Asparagin-Derivate, wurden bisher nicht als Substrate verwendet.

Es wurde nun gefunden, daß die Galactosyltransferase auch die Synthese der Glycoasparagine der Formel I und II erlaubt, wobei der nicht-galactosidische Teil zuvor durch eine Kombination bekannter Verfahren chemisch synthetisiert werden kann.

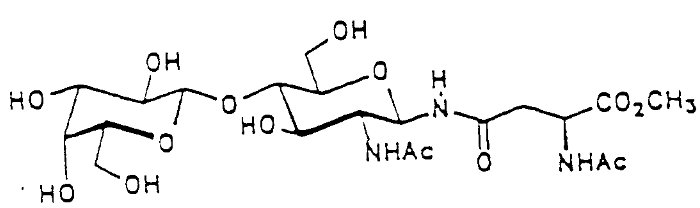

I

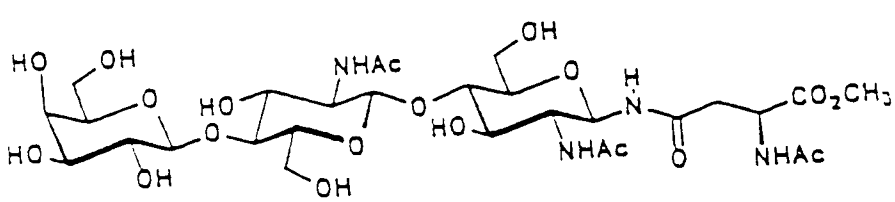

II

Gal-β(1→4)-GlcNAc-β(1→N)-Asn (I) tritt in seiner vollständig, also auch im Aminosäureteil deblockierten Form im Urin von Patienten auf, die an Aspartylglucosaminylurea leiden [R.J. Pollitt, K.M. Pretty, Biochem. J. 141 (1974) 141].

Gal-β(1→4)-GlcNAc-β(1→4)-GlcNAc-β(1-N)-L-Asn (II) findet sich in der Erythrozytenmembran. Es wurde die Vermutung ausgesprochen, daß es hier die Rezeptorstelle für Robinia-Lectin darstellt. [A.M. Leseney, R. Bourrillon, S. Kornfeld, Arch. Biochem. Biophys. 153 (1972) 831].

Die Erfindung betrifft somit ein Verfahren zur enzymatischen Herstellung der Verbindung der obengenannten Formel I oder der Verbindung der obengenannten Formel II, das dadurch gekennzeichnet ist, daß die Verbindung der Formel III oder die Verbindung der Formel IV

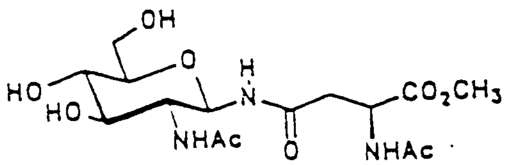

III

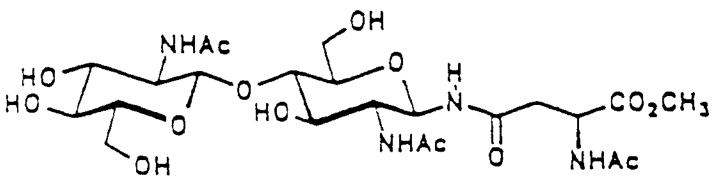

IV

in Gegenwart eines Galactosyldonors mit einer Galactosyltransferase inkubiert wird.

Nachfolgend wird die Erfindung im einzelnen erläutert und in ihren Patentansprüchen definiert.

Die Galactosyltransferase, z.B. EC 2.4.1.22, akzeptiert die Glycoasparagine der Formel III und IV als Substrate, die hierbei an der terminalen N-Acetylglucosamin-Einheit in β(1→4)-Stellung galactosyliert werden. Als Galactose-Donor benötigt das Enzym Uridin-5′-diphosphat-galactose (UDP-Gal). Dieser Nucleotidzucker ist jedoch sehr teuer und wird daher günstiger in situ aus Uridin-5′-diphosphat-glucose (UDP-Glc) in einer Reaktion erzeugt, die von dem Enzym Uridin-5′-diphosphat-galactose-4-epimerase (EC 5.1.3.2 ) katalysiert wird.

Zur Herstellung der Verbindung I oder II werden die genannten Ausgangssubstrate III bzw. IV zusammen mit einem Galactosyldonor, wie z.B. UDP-Gal, in wäßriger Lösung mit Galactosyltransferase behandelt. Die Ausgangssubstrate und der Galactosyldonor werden jeweils in äquimolarer Konzentration eingesetzt, bevorzugt in 10 bis 15 mmolarer Menge. Käufliche Galactosyltransferase kann in löslicher oder immobilisierter Form eingesetzt werden. Wenn immobilisiert wird, ist Kieselgel als Träger, insbesondere aus Kostengründen bevorzugt. Die Reaktion wird vorteilhaft in einer gepufferten Lösung durchgeführt. Als Puffer wird insbesondere Natriumcacodylat verwendet. Die Inkubationstemperatur kann entsprechend dem Temperatur/Aktivitätsbereich des Enzyms in weiten Bereichen schwanken. Bevorzugt wird die Reaktion bei 32-37 °C durchgeführt.

Wie schon oben erwähnt, kann die erfindungsgemäße Reaktion in einem sogenannten "Eintopfverfahren" mit der Herstellung von UDP-Gal aus UDP-Glc mit Hilfe von Uridin-5′-diphosphatgalactose-4-epimerase begonnen werden. Unter einem Eintopfverfahren wird verstanden, daß alle beteiligten Substrate und alle beteiligten Enzyme zusammen in einen Reaktionsansatz gegeben werden. Natürlich können die genannten Reaktionen, die Herstellung von UDP-Gal aus UDP-Glc und die Herstellung der Verbindungen I oder II, auch nacheinander durchgeführt werden. Ein Eintopfverfahren erwies sich jedoch, unter Einhaltung der oben beschriebenen Reaktionsparameter für den gesamten Ansatz, als wirtschaftlicher.

Die Detektion des Reaktionsverlaufs erfolgt dünnschichtchromatographisch. Die Abtrennung der Endprodukte wird nach Entfernung der Nucleotidphosphate mit Ionenaustauschchromatographie, wie z.B. an Dowex 1-X8 (Gegenion: $Cl^-$), und Gelchromatographie mit beispielsweise Bio-Gel P2 durchgeführt und gelingt in 25 - 35%iger Ausbeute.

Die Galactosylakzeptoren III und IV können, in Anlehnung an die Arbeiten verschiedener Autoren chemisch synthetisiert werden [D.E. Cowley, L. Hough, C.M. Peach, Carbohydr. Res. 19 (1971) 231; M. Spinola, R.W. Jeanloz, J. Biol. Chem. 245 (1970) 4158; H. Kunz, H. Waldmann, Angew. Chem. 97 (1985) 885].

Im Falle des Aspartyl-N-acetylglucosamins III wird N-Acetylglucosamin mit Acetylchlorid in das peracetylierte α-Chlorid übergeführt, welches anschließend phasentransferkatalytisch in Wasser/Chloroform zum β-Azid umgesetzt wird. Nach Reduktion mit Wasserstoff über Palladium zum β-Amin kann in einer Ethyl-2-ethoxy-1,2-dihydrochinolin-1-carboxylat (EEDQ)-unterstützten Kondensation mit N-Acetylasparaginsäure-α-methylester gekoppelt werden. Nach De-O-acetylierung mit Natriummethanolat wird der Galactosylakzeptor der Formel III erhalten, der vor der enzymatischen Galactosylierung noch gelchromatographisch gereinigt werden muß.

Zur Synthese des Aspartylchitobiosylamins IV wird zunächst Chitin acetolytisch in ein Oligomerengemisch gespalten, aus dem sich durch Flashchromatographie das Chitobioseoctaacetat isolieren läßt. Dieses wird durch Suspension in Acetylchlorid und Einleiten von gasförmigem Chlorwasserstoff in das peracetylierte α-Chitobiosylchlorid übergeführt. Entsprechend der Synthese der Verbindung der Formel III wird das α-Chlorid mit Natriumazid in das β-Azid übergeführt, zum β-Amin reduziert, mit N-Acetylasparaginsäure-α-methylester gekoppelt und zum Schluß de-O-acetyliert. Das so erhaltene Aspartylchitobiosylamin der Formel IV kann nach gelchromatographischer Reinigung für die beschriebene enzymatische Galactosylierung eingesetzt werden.

Im folgenden wird die Erfindung mit Hilfe von Beispielen weitergehend erläutert.

Beispiel

1. Vorschrift zur Galactosylierung der Glycosylasparagine III und IV

a. Die Reaktion wird in Natriumcacodylatpuffer (25 mM, pH 7,7) in Anwesenheit von Manganchlorid (5 mM) durchgeführt. Nach Zugabe von UDP-Galactose (13 mM), Glycosylasparagin (III bzw. IV, 13 mM), Rinderserumalbumin (1 mg/ml) und Desoxygenierung mit Stickstoff werden Galactosyltransferase (EC 2.4.1.22, 10 U) zugegeben. Nach zwei Tagen bei 37 °C wird mit Anionenaustauscher (Dowex 1-X8, Chlorid-Form) aufgearbeitet und nachfolgend durch Gelchromatographie an Bio-Gel P2 gereinigt.

b. Die Reaktion wird in Natriumcacodylatpuffer (25 mM, pH 7,7) in Anwesenheit von Manganchlorid (5 mM) durchgeführt. Nach Zugabe von UDP-Glucose (13 mM), Glycosylasparagin (III bzw. IV, 13 mM), Rinderserumalbumin (1 mg/ml) und Desoxygenierung mit Stickstoff werden Galactosyltransferase (EC 2.4.1.22, 1 U) und UDP-Galactose-4-Epimerase (EC 5.1.2.2, 10 U) zugegeben. Nach zwei Tagen bei 37 °C wird mit Anionenaustauscher (Dowex 1-X8, Chlorid-Form) aufgearbeitet und nachfolgend durch Gelchromatographie an Bio-Gel P2 gereinigt.

1.1) 2-Acetamido-4-O-(β-D-galactopyranosyl)-1-N-(N-acetyl-1-methyl-4-L-aspartyl)-2-desoxy-β-D-glucopyranosylamin (I)

| | | | |
|---|---|---|---|
| Ansatz: | 64,5 mg (100 µmol) UDP-Gal | | |
| | 39 mg (100 µmol) der Verbindung III | | |
| DC-Kontrolle: | Laufmittel | n-Propanol/Essigsäure/Wasser | 85:12:3 |
| | $R_f$-Werte: | GlcNAc-Asn (III) | 0,22 |
| | | Gal-GlcNAc-Asn (I) | 0,08 |

Ausbeute: 20 mg (35 %)

Ausgewählte $^1$H-NMR-Daten (300 MHz, $D_2O$): $\delta$ = 5,06 (d, 1H, H-1), 4,40 (d, 1H, H-1'), 2,95 (dd, 1H, β-Asn), 2,82 (dd, 1H, β'-Asn); $J_{1,2}$ = 9,4 $J_{1',2'}$ = 7,6, $J_{\alpha\cdot\beta}$ = 5,55, $J_{\beta,\beta'}$ = 17,1, $J_{\alpha\cdot\beta'}$ = 7,63 Hz.

1.2) 2-Acetamido-4-[2-acetamido-2-desoxy-4-(β-D-galactopyranosyl))-β-D-glucopyranosyl]-1-N-(N-acetyl-1-methyl-4-L-aspartyl)-2-desoxy-β-D-glucopyranosylamin (II)

Ansatz: 32,0 mg (50 µmol) UDP-Gal
29,0 mg (50 µmol) der Verbindung IV

DC-Kontrolle: Laufmittel n-Butanol/Essigsäure/Wasser 2:4:1

| | | |
|---|---|---|
| $R_F$-Werte: | GlcNAc-GlcNAc-Asn (IV) | 0,31 |
| | Gal-GlcNAc-GlcNAc-Asn (II) | 0,19 |

Ausbeute: 10 mg (25 %)

Ausgewählte $^1$H-NMR-Daten (330 MHz, $D_2O$): $\delta$ = 5,09 (d, 1H, H-1), 4,62 (d, 1H, H-1'), 4,47 (d, 1H, H-1''), 2,06, 2,02, 2,01 (je s, je 3H, je NAc);
$J_{1,2}$ = 9,6 Hz, $J_{1',2'}$ = 8,1 Hz, $J_{1'',2''}$ = 7,7 Hz.

**Patentansprüche**

1. Verfahren zur enzymatischen Herstellung der Verbindungen der Formel I oder II,

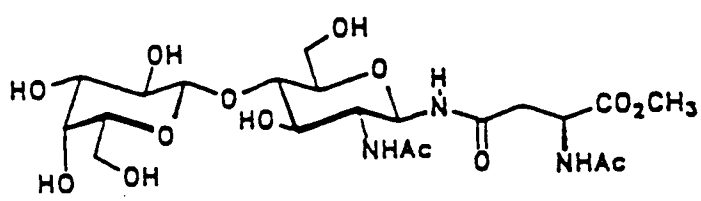

I

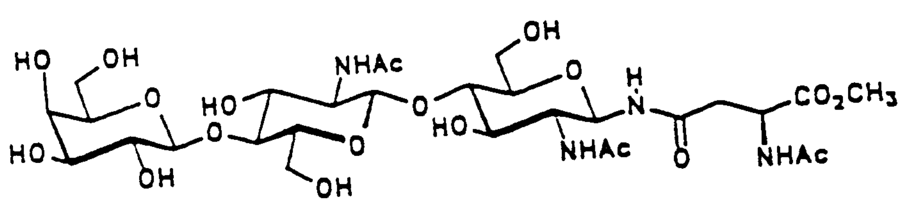

II

dadurch gekennzeichnet, daß die Verbindung der Formel III oder die Verbindung der Formel IV

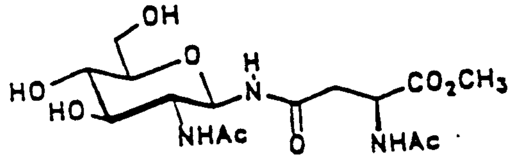

III

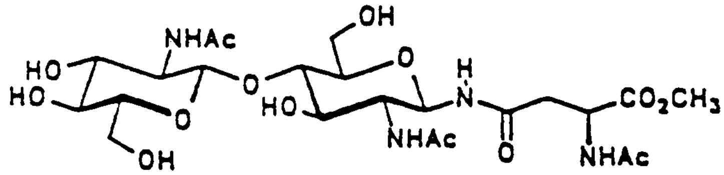

IV

in Gegenwart eines Galactosyldonors mit Galactosyltransferase inkubiert wird.

2. Verfahren nach Anspruch 1, dadurch gekennzeichnet, daß als Galactosyldonor UDP-Gal verwendet wird.

3. Verfahren nach Anspruch 1 oder 2, dadurch gekennzeichnet, daß UDP-Gal aus UDP-Glc unter Katalyse der UDP-Galactose-4-Epimerase synthetisiert wird.

4. Verfahren nach einem oder mehreren der Ansprüche 1 bis 3, dadurch gekennzeichnet, daß die Reaktion in einer gepufferten Lösung durchgeführt wird.

5. Verfahren nach einem oder mehreren der Ansprüche 1 bis 4, dadurch gekennzeichnet, daß die Reaktion bei 32 bis 37 °C durchgeführt wird.

**Claims**

1. A process for the enzymatic preparation of compounds of the formula I or II

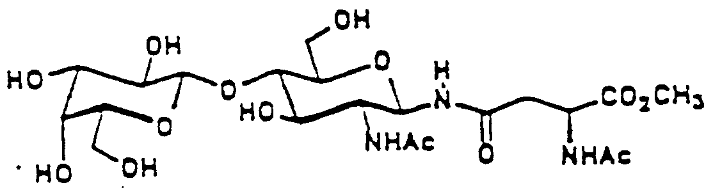

I

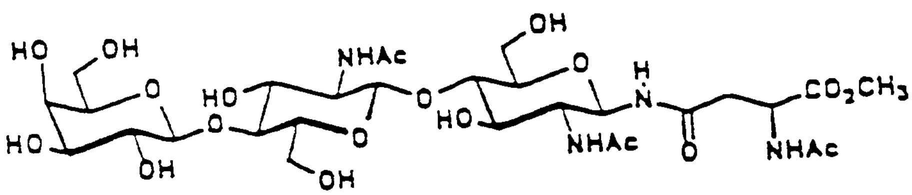

II

which comprises incubating the compound of the formula III or the compound of the formula IV

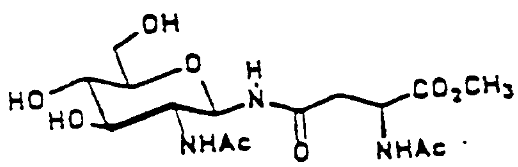

III

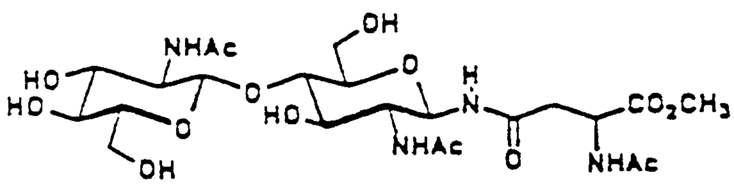

IV

with galactosyltransferase in the presence of a galactosyl donor.

2. The process as claimed in claim 1, wherein UDP-Gal is used as galactosyl donor.

3. The process as claimed in claim 1 or 2, wherein UDP-Gal is synthesized from UDP-Glc with catalysis by UDP-galactose 4-epimerase.

4. The process as claimed in one or more of claims 1 to 3, wherein the reaction is carried out in a buffered solution.

5. The process as claimed in one or more of claims 1 to 4, wherein the reaction is carried out at 32 to 37 ° C.

**Revendications**

1. Procédé pour la préparation enzymatique du composé de formule I ou du composé de formule II,

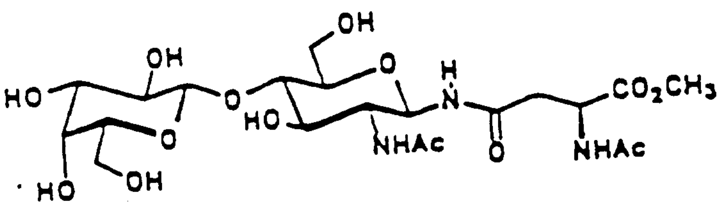

I

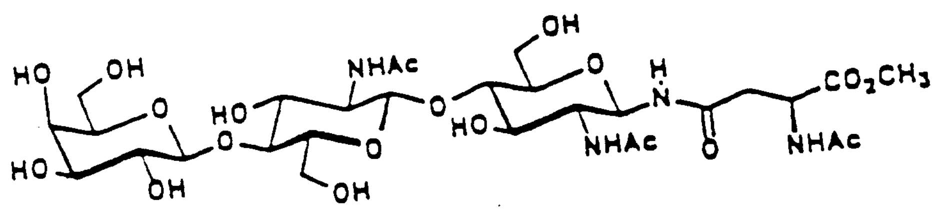

II

caractérisé en ce qu'on fait incuber avec une galactosyltransférase le composé de formule III ou le composé de formule IV suivants,

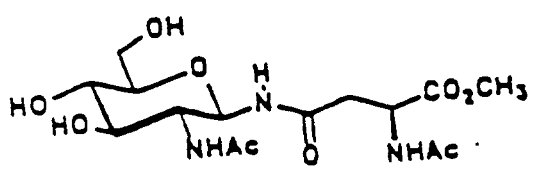

III

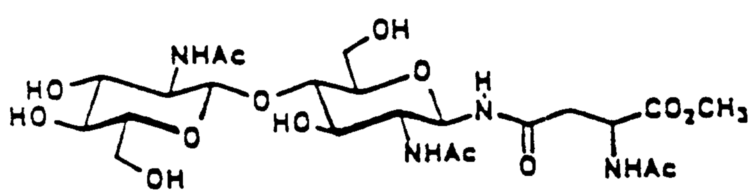

IV

en présence d'un donneur de galactosyle.

2. Procédé selon la revendication 1, caractérisé en ce qu'on utilise UDP-Gal comme donneur de galactosyle.

3. Procédé selon la revendication 1 ou 2, caractérisé en ce que UDP-Gal est synthétisé à partir de UDP-Glc, par catalyse par l'UDP-galactose-4-épimérase.

4. Procédé selon l'une ou plusieurs des revendications 1 à 3, caractérisé en ce que la réaction s'effectue dans une solution tamponnée.

5. Procédé selon l'une ou plusieurs des revendications 1 à 4, caractérisé en ce que la réaction a lieu à une température comprise entre 32 et 37 °C.